(19) Europäisches Patentamt
European Patent Office
Office européen des brevets

(11) EP 2 377 529 A1

(12) EUROPEAN PATENT APPLICATION
published in accordance with Art. 153(4) EPC

(43) Date of publication:
**19.10.2011 Bulletin 2011/42**

(21) Application number: **09831476.8**

(22) Date of filing: **10.12.2009**

(51) Int Cl.:
***A61K 31/352*** *(2006.01)* ***A61K 9/00*** *(2006.01)*
***A61K 9/08*** *(2006.01)* ***A61P 9/10*** *(2006.01)*

(86) International application number:
**PCT/CN2009/075456**

(87) International publication number:
**WO 2010/066199 (17.06.2010 Gazette 2010/24)**

(84) Designated Contracting States:
**AT BE BG CH CY CZ DE DK EE ES FI FR GB GR HR HU IE IS IT LI LT LU LV MC MK MT NL NO PL PT RO SE SI SK SM TR**

(30) Priority: **11.12.2008 CN 200810185559**

(71) Applicants:
- **CSPC Zhongqi Pharmaceutical Technology (Shijiazhuang) Co., Ltd.**
  **Hebei 050051 (CN)**
- **Institute of Mataria Medica, Chinese Academy of Medical Sciences**
  **Beijing 100050 (CN)**

(72) Inventors:
- **DU, Guanhua**
  **Beijing 100050 (CN)**
- **WANG, Jinxu**
  **Hebei 050051 (CN)**
- **WU, Song**
  **Beijing 100050 (CN)**
- **SHI, Ying**
  **Hebei 050051 (CN)**
- **GAO, Mei**
  **Beijing 100050 (CN)**
- **LI, Yingui**
  **Hebei 050051 (CN)**
- **QI, Yan**
  **Beijing 100050 (CN)**
- **SHEN, Dongmin**
  **Hebei 050051 (CN)**
- **GUANG, Hongmei**
  **Beijing 100050 (CN)**
- **LIU, Haili**
  **Hebei 050051 (CN)**
- **LIU, Rui**
  **Beijing 100050 (CN)**
- **FENG, Xiaolong**
  **Hebei 050051 (CN)**

(74) Representative: **Dorff, Gernot Peter Roderich**
**Eisenführ, Speiser & Partner**
**Postfach 10 60 78**
**28060 Bremen (DE)**

# (54) USE OF RACEMATES OF PINOCEMBRIN IN PREPARING MEDICAMENTS FOR TREATING STROKE

(57) Use of a racemate of pinocembrin, a racemate of pinocembrin salt, a racemate of pinocembrin precursor or a racemate of pinocembrin hydrate in manufacture of a medicament for prophylaxis and treatment of stroke. Particularly, use of pinocembrin racemate in manufacture of a medicament for treatment of acute ischemic stroke.

EP 2 377 529 A1

## Description

The present application claims the priority of Chinese Patent Application No. 200810185559.0 as filed on December 11, 2008 and titled with "Use of pinocembrin in preparing a medicament for treating stroke", and the disclosure of which is incorporated herein by reference.

## Technical Field

The present invention relates to the use of pinocembrin racemate in preparing a medicament for treatment and prophylaxis of stroke.

## Background Art

(S)-pinocembrin of Formula II, which chemical name is (S)-2,3-dihydro-5,7-dihydroxy- 2-benyl-4H-1-benzopyran-4-ketone, is a kind of water insoluble flavonone and a natural compound extracted from propolis. In addition, this compound is also found in extractive of a plurality of plants such as Helvetic five-leaved pine, leave of eucalyptus and acacia gum.

Since 1980s, researches all over the world discovered a plurality of pharmacological activities of (S)-pinocembrin, including antibiotic, antivirus, antioxidant and anti-inflammation effects. Chinese Academy of Medical Sciences first disclosed that (S)-pinocembrin has the function of inhibiting vasoconstriction caused by a plurality of factors and protecting damage of many kinds of nerve cells. Details can be found in CN200410037860.9, which disclosed the use of (S)-pinocembrin in treating cerebral ischemia, sequelae of cerebral ischemia, and diseases associated with damage and functional changes of nerve cells, and the mechanism thereof for protecting ischemic brain tissue and nerve cells mainly by inhibiting Calpain, NO and CRP and activating the expression of heat shock protein in the brain. Resumptively, (S)-pinocembrin can treat cerebral infarction. Moreover, Rui Liu etc. in Chinese Academy of Medical Sciences disclosed (S)-pinocembrin had the function of protecting the damaged nerve due to brain ischemic reperfusion or similar situations by the test of ischemia, hypoxia and apoptosis of brain tissue caused by brain ischemic reperfusion model (Pinocembrin protects rat brain against oxidation and apoptosis induced by ischemia-reperfusion both in vivo and in vitro. Brain Res. 2008 Jun 24;1216:104-15.).
Yonghao Cheng etc. disclosed synthesis of pinocembrin racemate (Formula □) (Yonghao Cheng, Yabo Duan, Yan Qi etc., Synthesis of 5,7-dihydroxy-flavanone, Chemical Reagents. 2006, 28(7):437).

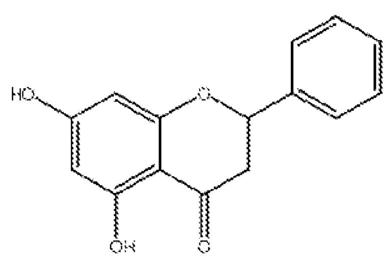

I

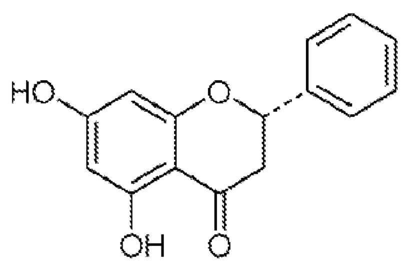

II

It is known from the prior art that racemate has no direct or certain relationship to efficacy. Overall, their relationship can be reduced to the following several situations: racemate has better effect compared to enantiomers; enantiomers have equal or close activities; enantiomers have activities of different strength; enantiomers have opposite activities; enantiomers have activities of different types. Then we amplify the points by the following examples.

1. Enantiomers are mutually synergistic, so racemate has better effect compared to enantiomers.

As a $\alpha$-receptor agonist, levo-isomer of dobutamine affects $\beta$-receptor slightly. Similarly, as a $\beta$-receptor agonist, dextral-isomer of dobutamine affects $\alpha$-receptor slightly. Administration by racemate will have an effect of increasing myocardial contractility but not speeding up the heart rate or raising blood pressure;

As an antihistamine, efficacy of isothipendyl racemate by oral administration is 1.4 times better than that of (-)-isomer, and 2.5 times better than that of (+)-isomer by oral administration. The reason why racemate has better efficacy is possibly that one isomer changes absorption of the other so that the bioavailability of the latter is raised, or that one isomer depresses the metabolic rate of the other so that the action time of the latter is extended.

2. The enantiomers have equal activities.

Promethazine as an antihistamine has one chiral molecule. Since its receptors have no selectivity to medicament enantiomers, its two enantiomers have the same pharmacologic actions and show the same strengths ofpharmacological activities.

3. Enantiomers have activities of different strengths.

Propranolol as a β-receptor antagonist mainly depends on its levo-isomer to exhibit antagonistic activity, because its levo-isomer has the same configuration to the β-receptor agonist and can selectively bind to β-receptor, while its dextral- isomer can not.

In another extreme example, L-methyldopa as an antihypertensive drug has pharmacological activities, while D-methyldopa has no activity.

4. Enantiomers have opposite activities.

As to Bay k8644, a nifedipine structural analogue, its dextral-enantiomer is a calcium antagonist, whereas its levo-enantiomer is a calcium agonist. The two enantiomers have completely opposite activities.

5. Enantiomers have pharmacological activities of different types.

D-enantiomer of propoxyphene has a strong analgesic activity which is 6 times of the activity of L-enantiomer, but has no antitussive effect. On the contrary, L-enantiomer of propoxyphene has strong antitussive effect. (Medicinal Chemistry, edited by Wensheng Ji, Anliang Li, Higher Education Press: 25-26)

It is further known from the above examples that according to the known pharmacological activity of (S)-pinocembrin, it can not be determined without experiments whether the racemate of pinocembrin or (R)-pinocembrin has the same pharmacological activity and effect of (S)-pinocembrin. The following documents are retrieved in the prior art.

Xiaoming Zhu, etc. disclosed that pinocembrin could expand the vascular of thoracic aorta by endothelium-dependent mechanism and endothelium-independent mechanism (Zhu XM, Fang LH, Li YJ, Du GH. Endothelium-dependent and Endothelium-independent relaxation induced by pinocembrin in rat aortic rings. Vascul Pharmacol. 2007; 46(3): 160).

Mei Gao, etc. disclosed that pinocembrin could improve the glutamate-induced cell injury and apoptosis and also reduce the probability of apoptosis, which provide the evidence of neuro-protective activity of pinocembrin to its effect of anti-cerebral-ischemia (Mei Gao, Wen-cui Zhang, Qing-shan Liu, Juan-juan Hu, Geng-tao Liu, Guan-hua Du. Pinocembrin prevents glutamate-induced apoptosis in SH-S Y5Y neuronal cells via bax/bcl-2 ratio decrease. Eur J Pharmacol. 2008, 591 (1-3):73-9).

Mei Gao, etc. disclosed that pinocembrin could protect cerebral neurovascular of permanent cerebral ischemia in rats (Acute neurovascular unit protective action of pinocembrin against permanent cerebral ischemia in rats. J Asian Nat Prod Res. 2008 May-Jun; 10(5-6): 551-8). It was the first time that the protective action of pinocembrin against permanent focal cerebral ischemia was proved. Its mechanism is still in research.

Hongmei Guang etc. obtained the model of insufficient blood supply to brain by bilateral carotid artery ligation and detected the cognitive function by Morris water maze test. It was disclosed that pinocembrin could bring about improvement in cognitive dysfunction in rats caused by insufficient cerebral blood supply and its mechanism was that pinocembrin could protect the structure and function of mitochondria (Protections of pinocembrin on brain mitochondria contribute to cognitive improvement in chronic cerebral hypoperfused rats. Eur J Pharmacol. 2006 Aug 7; 542 (1-3): 77-83).

The above documents disclose in some extent the pharmacological activities of pinocembrin, but are limited to the action of vascular expansion and neurovascular protection.

It is known in the prior art that acute cerebral ischemic stroke (cerebral ischemic apoplexy) has a high morbidity, a high mortality and a high rate of disability. At present, the most effective treatment is thrombolysis. The earlier the thrombolysis is performed, the better the treatment effect is. The clinical experiences in the past many years showed that the rescue of stroke patients needs to race against time. Once cerebral artery is blocked, the cerebral cells in ischemic region will start up a cascade electrochemical chain reaction quickly, and will produce abundant free radicals, arouse influxing of calcium ions and overloading of intracellular calcium ions and final lead to irreversible damage in brain tissue. Therefore, the time from onset to rescue of stroke should be minimized as much as possible.

In clinical practice, cerebral ischemic stroke can be divided into the following types: ultra-early stage, within 6 hours of onset; early stage, during 6-72 hours of onset; acute later stage, during 72 hours-1 week of onset; recovery stage, 1 week after onset. During the ultra-early stage of ischemic stroke, cerebral infarction has not engendered yet.

If normal blood supply can be recovered promptly and harmful metabolites in ischemic tissues can be removed, the patients will have great opportunity to recover completely. So treatment in ultra-early stage will be the best opportunity and will get a good result. Up to early stage of ischemic stroke, persistent ischemia, hypoxia and especially blood-brain barrier being damaged cause that the regions of central infarction tend to form. As compared with ultra-early stage treatment, the treatment in this stage loses a lot of therapeutic value.

In 1996, recombinant tissue plasminogen activator (tPA) passed through the clinical validation of FDA and was approved for use within 3 hours after onset of acute ischemic stroke. This is the only medicament proved effective on treatment of ischemic stroke. Restricted by therapeutic time window, 95% of acute stroke patients abroad can not be treated timely by thrombolysis and in China the number of patients obtaining thrombolysis therapy is less than 1%.

Over the last decade, research for neuroprotective agents becomes a hot spot on stroke treatment. But in 114 stroke trials (up to 49 neuroprotective agents involved) all over the world, few trials were proved successful. This means that still no neuroprotective agent is proved safe and effective on treatment of acute ischemic stroke currently.

From the above analysis, it can be seen that, except tPA, drugs for effectively treating stroke are still in need in clinical practice, and even tPA is restricted by therapeutic time window of only 3 hours.

## Contents of the Invention

By experiments, the present invention surprisingly disclosed that pinocembrin racemate can be used to treat stroke. Compared with (S)-pinocembrin, pinocembrin racemate has a longer therapeutic time window (about 6 hours) and will still have therapeutic effect during a longer time period after onset of stroke. The extension of therapeutic time window brings more opportunities for patients of ischemic stroke. Animal experiments show that pinocembrin racemate has significant therapeutic effects.

One object of the present invention is to provide a use of a racemate of pinocembrin, a racemate of pinocembrin salt, a racemate of pinocembrin precursor or a racemate of pinocembrin hydrate in manufacture of a medicament for treating stroke.

The terminology "racemate" means an equimolar mixture of a chiral molecule with optical activity and its enantiomer, which is formed by mixing equivalent amounts of two molecules with opposite rotations and equivalent rotary powers so that their rotations are neutralized due to their intermolecular action.

The salt of pinocembrin is a pharmaceutically acceptable salt of pinocembrin, such as hydrochloride, sulfate and citrate, etc.

The precursor of pinocembrin is a prodrug of pinocembrin, that is, a compound that can produce pharmacological activities only after changed into pinocembrin by conversion in vivo.

Preferably, the stroke is acute ischemic stroke.

It is found by experiments that:

1) Racemate of pinocembrin can be used to treat stroke;
2) Racemate of pinocembrin can improve behavioral changes caused by acute ischemic stroke;
3) Racemate of pinocembrin can improve decrease in cerebral blood flow caused by acute ischemic stroke;
4) Racemate of pinocembrin can decrease volume of cerebral infarct caused by acute ischemic stroke;
5) Racemate of pinocembrin can improve cerebral edema caused by acute ischemic stroke;
6) Racemate of pinocembrin can improve energy metabolism caused by acute ischemic stroke;
7) Racemate of pinocembrin can improve acute inflammation caused by acute ischemic stroke;
8) Racemate of pinocembrin can protect nerve cells from damage caused by acute ischemic stroke.

Therefore, the present invention also relates to a pharmaceutical composition of pinocembrin racemate, which consists of a racemate of pinocembrin, a racemate of pinocembrin salt, a racemate of pinocembrin precursor or a racemate of pinocembrin hydrate and a pharmaceutically acceptable excipient.

The composition of the present invention can be suitable for oral, transdermal, muscular, intravenous or mucosal administration.

The composition of the present invention can be a solid or liquid preparation, which can be prepared by a conventional method.

The composition of the present invention can be a liquid preparation, preferably an injection. The injection is prepared by encapsulating pinocembrin racemate with cyclodextrin or its derivatives, and then adding a suitable excipient.

The present invention is also to provide a use of (R)-pinocembrin, its salts, its precursors or its hydrates in manufacture of a medicament for treatment and prophylaxis of stroke, in which the stroke is ischemic stroke or hemorrhagic stroke.

Pinocembrin racemate used in present invention has the merit of low toxicity and can be used to treat acute ischemic stroke.

## Brief Description of the Drawings

Figure 1 shows the effects of pinocembrin racemate on infarct volume.
Figure 2 shows the effects of pinocembrin racemate on expression of TNF-α, IL-1β, ICAM-1, VCAM-1, iNOS and AQP-4 in ischemic cerebral tissues after MCAO for 24h; in which

A shows the effects of DL0108 on expression of TNF-α, IL-1β, ICAM-1, VCAM-1, iNOS and AQP-4;
B is a statistic chart that quantifies the expression of TNF-α, IL-1β, ICAM-1, VCAM-1, iNOS and AQP-4 with β-actin as internal standard.
Figure 3 is a Nissl staining figure (200×) of CA1 area in cortex and hippocampus, in which

A: sham operation group;
B: model group;
C: nimodipine 3mg/kg group;
D: DL0108 (pinocembrin racemate) 1mg/kg group;
E: DL0108 (pinocembrin racemate) 3mg/kg group;
F: DL0108 (pinocembrin racemate) 10mg/kg group.

## Specific Modes for Carrying out the Invention

The present invention is illustrated specifically in the following examples.

Example 1: Effects of prophylaxis and treatment on stroke in spontaneously hypertensive rats of stroke prone (SHRSP)

1. Test drugs: pinocembrin racemate for injection, (R)-pinocembrin for injection and (S)-pinocembrin for injection, which are provided by New Drug Development Laboratory of Drug Research Institute of the Chinese Academy of Medical Sciences (Batch number: 20050601, content: 2.36%), and prepared by the method disclosed in CN200810084682.3, i.e., forming an inclusion complex of pinocembrin racemate or pinocembrin enantiomers with cyclodextrin or its derivatives, and dissolving the complex in a physiological saline when used. Nimodipine as a positive control drug was purchased from Bayer Company (Germany).
2. Experimental animals and grouping:

Experimental animals: 110 SHRSP rats and 10 normal Wistar rats of 6 weeks.
Experimental grouping: Wistar rats were normal group; SHRSP rats were grouped as a model group, a nimodipine group (3mg/kg), groups of low, middle and high dose of pinocembrin racemate (3, 10 and 30 mg/kg/d, respectively), groups of low, middle and high dose of (R)-pinocembrin (which doses were identical to those of pinocembrin racemate groups, respectively), and groups of low, middle and high dose of (S)-pinocembrin (which doses were identical to those of pinocembrin racemate groups, respectively).

3. Experimental method:

Except the rats of the normal group, all SHRSP rats drank 1% sodium chloride solution everyday. The drugs were administered in prescriptive doses respectively for the rats of the nimodipine group (3mg/kg), the groups of low, middle and high dose of pinocembrin racemate (3, 10 and 30 mg/kg/d, respectively), the groups of low, middle and high dose of (R)-pinocembrin (which doses were identical to those of pinocembrin racemate groups, respectively), and the groups of low, middle and high dose of (S)-pinocembrin (which doses were identical to those of pinocembrin racemate groups, respectively). Physiological saline of same volume was administered for the model group. The administration was continued for 14 days after the onset of stroke.

4. Standard of stroke onset:

☐ Appearance of twitch on one limb, double forelimbs or whole body; ☐ appearance of hemiplegia or general paralysis, abdomen attaching to the ground and being unable to stand up; ☐ to the more serious, appearance of

systemic fine tremor, groveling, flaccid paralysis, even death. Besides the symptoms in the above terms, irritation (jumping or leaping up) could be observed in some animals. Once any one of the above symptoms was observed, the onset of stroke was determined, and the time of onset of every animal was recorded.

5. Specimen collection:

1) After the last dose, the rats were fasted for 6 hours, and blood samples were collected by blood sampling in eyeground. After decollation, the whole brains of the rats were detached at ice trays for use.
2) Blood of 3mL was dropped rapidly in a cuvette with 1mL 0.074% heparin and mixed. The whole blood viscosity, plasma viscosity, red blood cell (RBC) aggregation index and RBC deformation index were determined with a hemorheology detector according to its specifications.
3) Blood of 2mL was dropped rapidly in a cuvette with 0.2mL 0.13mol/L Sodium citrate) and mixed. The mixture was centrifuged for 10 min at 1000rpm and platelet aggregation ratio was determined with a platelet aggregation meter according to its specifications.
4) Blood of 1mL was dropped rapidly in a cuvette with 0.1mL 0.109mol/L Sodium citrate and mixed. The mixture was centrifuged for 15 min at 3000rpm and plasma fibrinogen in liquid supernatant was determined by euzymelinked immunosorbent assay (ELISA).
5) Blood of 2mL was dropped rapidly in a cuvette with 30μL 7.5% EDTA-2Na and 40μL aprotinin and mixed. After centrifuged for 5 min at 3000rpm, plasma ET-1 in liquid supernatant was determined by isotope radioimmunoassay.
6) The whole brain tissues were weighed one by one, dried in a vacuum over at 110±5 □ for 48 hours, and then weighed again.

6. Observable indexes:
1) Observing body status of animals of every groups after modeling: including body appearance, mental state, responsiveness, posture in movement, spontaneous activity, amounts of water and food, and body weight etc.
Scores of neurological symptoms after stroke onset:

| Score | SYMPTOMS |
|---|---|
| 0 | Normal |
| 1 | less movement or mild irritation |
| 2 | twitch in one forelimb or head, being inactive in the corner of cage or irritated excitement (appearance of jumping or leaping up) |
| 3 | paralysis in both forelimb and hindlimb by one side or in one forelimb, body skew, difficulty in walking |
| 4 | abdomen attaching to ground and unable to stand, quadriplegia and fine tremor in whole body |

After stroke onset, the scores during 14 days were recorded for every animal, and the scores in the treated groups are compared with scores in the control group.
2) Hemorheology: including whole blood viscosity, plasma viscosity, RBC aggregation index, RBC deformation index and platelet aggregation ratio.
3) Plasma fibrinogen
4) Plasma ET-1
5) Brain water content: according to the weights of whole brain tissue before and after taken into the vacuum oven, the data were applied to the following formula and calculated:

$$\text{Brain water content} = (\text{ wet weight} - \text{dry weight }) / \text{wet weight} \times 100\%$$

6. Statistical methods: the results were showed in the format of $\bar{x} \pm$ SD and the comparison between the data was assayed by one-way analysis of variance (ANOVA).
7. Experimental results:

1) Death of rats:

Totally 24 rats had died during the experiment. After analysis of pathological autopsy, it was found that 4 of them died of hemorrhagic stroke and other 20 rats died of ischemic stroke. Details can be seen in Table 1.

Table 1: Death of rats and analysis table

| GROUP | DOSE (mg/kg) | TIME OF STROKE ONSET (d) | NUMBER OF RATS | | CAUSES OF DEATH |
|---|---|---|---|---|---|
| | | | Before experiment | After experiment | |
| Normal | -- | -- | 10 | 10 | -- |
| Model | -- | 33.7±5.1 | 10 | 4 | hemorrhagic:1<br>ischemic stroke:5 |
| Nimodipine | 3 | 41.6±12.8* | 10 | 10 | -- |
| Pinocembrin racemate | 30 | 43.9±6.4* | 10 | 9 | Ischemic stroke:1 |
| | 10 | 42.7±7.2** | 10 | 10 | -- |
| | 3 | 40.1±8.7* | 10 | 10 | -- |
| (R)-pinocembrin | 30 | 41.8±6.9* | 10 | 7 | ischemic stroke:3 |
| | 10 | 39.8±7.8* | 10 | 7 | hemorrhagic stroke: 1<br>ischemic stroke:2 |
| | 3 | 37.7±8.4 | 10 | 9 | ischemic stroke:1 |
| (S)- pinocembrin | 30 | 41.5±5.8* | 10 | 7 | ischemic stroke:3<br>hemorrhagic stroke: 2 |
| | 10 | 37.6±7.3 | 10 | 6 | ischemic stroke: 2 |
| | 3 | 34.3±6.9 | 10 | 7 | ischemic stroke:3 |

Compared with the model group, *P<0.05, **P<0.01.

2) Mental state of the rats:

The rats in the normal group were normal in body condition, eating and movement.

From the beginning of the forth week, except the groups of high, middle dose of pinocembrin racemate and the nimodipine group, in other groups there had been constantly some rats attacked by stroke showing symptoms of irritation, paroxysmal convulsions, paralysis, heavy head, eating less, urinary incontinence, hair shaft and becoming thin. According to the above scoring standard of neurological symptoms after stroke onset, beginning with the day of stroke onset, the scores for the groups of high and middle doses of pinocembrin racemate decreased remarkably. Within 2-14 days after onset, their scores were stably below 1.6 and 2.3 respectively. This indicated that the high and middle dose of pinocembrin racemate had significant improvement effects in neurological function. The scores for the group of low dose of pinocembrin racemate and the group of high dose of (R)-pinocembrin not only were relatively high during the beginning several days but also were maintained at 2.5 or above for 14 days. This indicated that the low dose of pinocembrin racemate and the high dose of (R)-pinocembrin had no improvement effect. After onset of stroke, the scores of the nimodipine group were relatively low and were stably below 2.2 after 6 days.

3) Analysis of hemorheology

Table 2: Changes of hemorheologic indexes ($\overline{X}\pm S$)

| GROUP | DOSE (mg/kg) | whole blood viscosity | | plasma specific viscosity | RBC aggregation index | RBC deformation index |
|---|---|---|---|---|---|---|
| | | 20 (l/s) | 5 (l/s) | | | |
| normal | -- | 2.99±0.48 | 10.08±1.99 | 1.82±0.28 | 7.43±0.63 | 0.23±0.023 |
| model | -- | 5.90±0.67## | 15.49±3.06## | 2.75±0.42## | 14.67±1.13## | 0.104±0.019## |
| nimodipine | 3 | 4.64±0.92* | 11.84±2.44* | 1.98±0.29** | 8.69±1.01** | 0.213±0.03** |
| pinocembrin racemate | 30 | 3.89±0.28** | 10.95±1.30* | 1.99±0.28* | 7.84±0.63* | 0.222±0.028** |
| | 10 | 4.12±0.57* | 12.01±2.46* | 2.01±0.49 | 8.36±1.39** | 0.219±0.031* |
| | 3 | 4.66±0.34 | 14.81±2.13 | 2.17±0.28 | 8.84±0.63* | 0.183±0.028* |
| (R)-pinocembrin | 30 | 4.28±0.66* | 12.24±3.16* | 2.09±0.51 | 8.12±0.88** | 0.219±0.104* |
| | 10 | 4.45±0.71 | 14.39±1.84 | 2.30±0.63 | 8.25±2.41* | 0.184±0.100* |
| | 3 | 5.06±1.45 | 15.20±1.82 | 2.60±0.94 | 8.50±2.11* | 0.144±0.405 |
| (S)-pinocembrin | 30 | 4.96±1.26 | 14.90±3.32 | 2.47±0.15 | 12.50±2.11 | 0.120±0.405 |
| | 10 | 5.49±0.43 | 15.13±3.06 | 2.65±0.42 | 13.07±1.13 | 0.109±0.082 |
| | 3 | 5.78±0.06 | 15.39±1.20 | 2.79±0.42 | 14.45±1.13 | 0.101±0.030 |

Compared with the normal group, #P<0.05, ##p<0.01; Compared with the model group, *P<0.05, **P<0.01.

The results show that the model group exhibited significant differences (P<0.01) in the blood hemorheology indexes as compared with the normal group, which illustrated the state of high viscosity and high aggregation of blood of the model group. Compared with the model group, the groups of high, middle and low dose of pinocembrin racemate and the group of high dose of (R)-pinocembrin exhibited a decrease of blood agglomeration in different extents ( P<0.01, P<0.05), especially exhibited a decrease in whole blood viscosity and RBC aggregation index (P<0.01). However, (S)-pinocembrin showed no activity at all. Nimodipine had an effect of decreasing blood viscosity. Results are shown in Table 3.

Suggestion: pinocembrin racemate can decrease blood viscosity in a dose-dependent manner, and (R)-pinocembrin in high dose has the similar effect.

4) Platelet aggregation:

The results of resistance to platelet aggregation in the Table 3 indicated that the platelet aggregation ratio of the model group were higher than that of the normal group (P<0.01). Compared with the model group, the groups of high and middle dose of pinocembrin racemate and the group of high dose of (R)-pinocembrin exhibited a significant decrease in platelet aggregation ratio (P<0.01, P<0.05), while (S)-pinocembrin showed no activity.

Suggestion: pinocembrin racemate can resist platelet aggregation in a dose-dependent manner, and (R)-pinocembrin in high dose also has effect.

5) Plasma fibrinogen assay:

The content of plasma fibrinogen in the model group was significantly higher than that in normal group (P<0.01). Compared with the model group, the groups of high, middle and low dose of pinocembrin racemate and the group of high dose of (R)-pinocembrin exhibited a significant decrease in content of plasma fibrinogen (P<0.01) thereby changing the hypercoagulability condition, while (S)-pinocembrin had no activity. Specific results are shown in Table 3.

Table 3: Platelet aggregation change, plasma fibrinogen content, ET-1 content and brain water content

| Group | Dose (mg/kg) | Change of platelet aggregation | Plasma fibrinogen | ET-1 content | Brain water content |
|---|---|---|---|---|---|
| normal | -- | 19.4±2.6 | 2.55±0.12 | 163.6±21.6 | 78.34±0.62 |
| model | -- | 26.8±3.1## | 3.05±0.26## | 243±21.2## | 79.85±0.70# |

(continued)

| Group | Dose (mg/kg) | Change of platelet aggregation | Plasma fibrinogen | ET-1 content | Brain water content |
|---|---|---|---|---|---|
| nimodipine | 3 | 20.1±2.8** | 2.67±0.13** | 174.8±20.8** | 78.82±0.58* |
| pinocembrin racemate | 30 | 22.3±3.5** | 2.56±0.42** | 182.4+24.2* | 78.67±0.60* |
| | 10 | 23.8±2.9* | 2.62±0.15** | 209.2±16.7 | 79.08±0.67 |
| | 3 | 25.1±3.0 | 2.79±0.13* | 221.4±20.4 | 79.51±0.42 |
| (R)-pinocembrin | 30 | 23.9±6.1* | 2.74±0.47* | 198.1±35.4* | 79.00±0.14 |
| | 10 | 25.4±4.3 | 2.83±0.18 | 228.4±16.9 | 79.54±0.53 |
| | 3 | 25.9±1.7 | 2.99±0.13 | 235.4±35.2 | 79.55±0.55 |
| (S)-pinocembrin | 30 | 25.7±5.4 | 2.81±0.57 | 240.8±24.3 | 79.67±0.73 |
| | 10 | 26.5±4.1 | 2.99±0.61 | 240±16.8 | 79.75±0.81 |
| | 3 | 26.7±1.3 | 3.00±0.32 | 243±19.5 | 79.95±0.68 |

Compared with the normal group, $^{\#}$P<0.05, $^{\#\#}$p<0.01; Compared with the model group, *P<0.05 , **P<0.01.

6) Plasma ET-1

As showed in Table 3, the plasma ET-1 in the model group increased rapidly and had significant difference in comparison with the normal group (P<0.01). Compared with the model group, the plasma ET-1 contents in the group of high dose of pinocembrin racemate and the group of high dose of (R)-pinocembrin decreased significantly (P<0.01, P<0.05). The groups of other doses of pinocembrin racemate and (R)-pinocembrin and the groups of (S)-pinocembrin had no activity.

7) Determination of water content in dropsical brain:

As showed in Table 3, the brain water content in the model group increased markedly and had significant difference in comparison with the normal group (P<0.05). Compared with the model group, the brain water content in the group of high dose of pinocembrin racemate decreased significantly (P<0.05). Other groups had no remarkable activity.

8) Conclusion:

Compared with the model group, pinocembrin racemate can delay the time of stroke onset in dose-dependent manner (P<0.01). Pinocembrin racemate in low dose and (R)-pinocembrin in high dose can also delay the time of stroke onset (P<0.05). Nimodipine can delay the time of stroke onset (P<0.01). It is illustrated that pinocembrin has prophylactic effects on stroke, pinocembrin racemate is superior to (R)-pinocembrin, and (S)-pinocembrin in high, middle and low doses had no activity. The specific results can be seen in Table 1.

Pinocembrin racemate can dose-dependently increase survival ratio after stroke, which indicates pinocembrin racemate has the activity of treating stroke in SHRSP rats.

(R)-pinocembrin has this activity only when administered by high dose, and the activity is similar to that of nimodipine, which indicates that pinocembrin racemate has better activity than (R)-pinocembrin, while (S)-pinocembrin has no significant activity. Suggestions: 1) pinocembrin racemate has effects of prophylaxis and treatment on stroke; 2) (R)-pinocembrin also has effects of prophylaxis and treatment on stroke, although the effects are somewhat less; 3) the treatment effects of pinocembrin racemate are better than that of (R)-pinocembrin generally because (S)-pinocembrin without effects promoted the effects of (R)-pinocembrin.

Example 2: Effects on acute ischemic stroke caused by middle cerebral artery occlusion (MCAO)

Test drugs: pinocembrin for injection, provided by New Drug Development Laboratory of Drug Research Institute of the Chinese Academy of Medical Science (Batch number: 20050601, content: 2.36%), and prepared by the method

disclosed in CN200810084682.3, i.e., forming an inclusion complex of pinocembrin racemate with cyclodextrin or its derivatives, and dissolving the complex in a physiological saline when used. Nimodipine as the positive control drug was purchased from Bayer Company (Germany).

Experimental animals: 100 male SD rats with a body weight of 250-280g were purchased from the Experimental Animals Institute of the Chinese Academy of Medical Science. The rats were grouped randomly as a sham operation group, a model group, pinocembrin groups and a nimodipine group (3mg/kg).

Experimental model: the model of acute ischemic stroke was made by middle cerebral artery occlusion (MCAO).

The string occlusion method in middle cerebral artery as established by Zea Longa was adopted with proper improvements. 400 mg/kg 10% chloral hydrate was intraperitoneally injected in rats. The external carotid artery (ECA) was separated and ligated at the site where the artery emitted for about 0.8cm. The end of ECA closing to the heart was clipped by an artery clip. A 2mm V-shaped incision was made from ECA ligation to ECA bifurcation. A nylon line was inserted gently into CCA and then into internal carotid artery (ICA) through the bifurcation of ECA and ICA after loosening the artery clip. The nylon line was pushed into the brain through ICA and inserted in depth of 18.5±0.5mm when resistance was felt slightly. The nylon line should reach smaller anterior cerebral artery through the beginning of middle cerebral artery (MCA) and ICA could be ligated and sutured. Stump of the nylon line should be reserved with 1 cm outside the skin.

Rats in the sham operation group were only operated with preoperative anesthesia and vascular dissection without ligation and importing the line.

Statistical method: the results were showed in the format of $\overline{x} \pm SD$, and the comparison among the groups was performed by one-way analysis of variance (ANOVA).

Observed indexes and results:

1. Neurobehavioral examination (Bederson's score):

Neurobehavioral examination was performed before the animals were put to death. The rats were raised off the ground for about 1 chi (1 chi = 1/3 meter) to observe the state of two forelimbs. The rats were placed on the ground and were pushed by their shoulders to observe differences of their resistance. The rats were placed on the ground and were observed by the way of walking. Four-stage scoring method (0-5 scores) was adopted. The higher the score was, the more serious injury existed in their neurobehavior.

Bederson's scoring standard:

(1) If the rats acted completely normal, score 0 was recorded.
(2) When the rats were raised off the ground, if the rats rotated or retracted forelimb of surgery-contralateral side internally, score 1 was recorded.
(3) When the rats were placed on the ground and squeezed with the hand to examine the resistance of both sides, if the resistance of surgery-contralateral side declined, score 2 was recorded.
(4) When the rats were placed on the ground to observe the way of walking, if the rats circled around the surgery-contralateral side, score 3 was recorded.
(5) If the rats were injured too seriously to move by themselves, score 4 was recorded.

The results were shown in Table 4: the neurobehavioral score of the rats in the sham operation group was 0. The average neurobehavioral score of the rats in the model operation group was 3.4±0.6, in which most animals showed internal rotation or retraction of their forelimbs at surgery-contralateral side, a reduction of muscle stretching force at contralateral side, circling or occasionally circling, and were scored 3; a minority of animals showed only internal rotation of their forelimbs and reduction of resistance, and were scored 2; several animals showed severe symptoms and did move by themselves, and were scored 4.

In the pinocembrin racemate groups (3mg/kg, 10mg/kg, 30mg/kg), symptoms of nerve injury caused by ischemia in animals were improved significantly (P<0.05, P<0.01) showing a dose-effect relationship.

Table 4: Effects of pinocembrin racemate on neurological symptom scores (Bederson's scores) and infarct volumes ($\overline{X}\pm$ s, n=10)

| Group | Dose (mg/kg) | Bederson's score | Percentage of infarct volume (%) |
|---|---|---|---|
| sham operation | -- | $0.4\pm0.1$ | 0 |
| model | -- | $3.4\pm0.6^{\#\#}$ | $33.6\pm4.3^{\#\#}$ |
| Pinocembrin racemate | 3 | $2.4\pm0.7$* | $23.1\pm3.4$* |
| Pinocembrin racemate | 10 | $1.8\pm0.6$** | $21.4\pm2.1$* |
| Pinocembrin racemate | 30 | $1.5\pm0.3$** | $14.6\pm1.1$** |
| Nimodipine | 3 | $2.5\pm0.7$* | $16.7\pm1.3$** |

Compared with the sham operation group, $^{\#\#}p<0.01$; Compared with the model group, *P<0.05 , **P<0.01.

2. Determination of cerebral infarct volume:

After neurobehavioral scoring, the rats were put to death by decollation and their brain tissues were removed quickly and put into a refrigerator of -20˚C. 10 min later, the brain tissue was moved to an environment of room temperature. After the olfactory bulb, cerebellar and lower brain stem were resected, the brain tissue was cut into five continuous coronal slices with an interval of 2mm and was cut first on the connection midpoint of brain anterior pole and optic decussation, second on optic decussation, third on funnel handle and fourth on midpoint of funnel handle and thick leaves caudate nucleus. Then the brain slices were quickly put into 5ml solution containing 4% TTC and 0.1ml 1mol•$L^{-1}K_2HPO_4$ and incubated at a constant temperature of 37˚C and in condition of darkness for 30min. During the time period, the brain slices were tipped every 5 min. After TTC staining, normal tissue showed rose red color, while infracted tissue was not stained and showed white color. Every group of brain slices were put in order and photographed. The photographs were processed by an image analysis system and infarct area in every slice was calculated. The infarct volume of every slice was calculated by multiplying the infarct area by the thickness of slice (2mm), and the infarct volume was the sum of infarct volumes of all slices. The infarct volume was showed in percentage in hemisphere so as to eliminate the effects of cerebral edema.

Infarct volume (%) = (volume of surgery-contralateral hemisphere - infarct volume of surgery-homolateral hemisphere) / volume of surgery-contralateral hemisphere × 100%

The results showed that: after injured by cerebral ischemia, no cerebral infarction had been found in the sham operation group, while the infarct volume in the model group was $(33.6\pm4.3)$% (P<0.01). Compared with the model group, the infarct volumes in the pinocembrin racemate groups (3, 10, 30 mg/kg) were significantly reduced (P<0.01) and the infarct volumes were respectively $(23.1\pm3.4)$%, $(21.4\pm2.1)$% and $(14.6\pm1.1)$%. The infarct volume in the nimodipine group was $(16.7\pm1.3)$% and significantly different from that of the model group (P<0.01). The results were shown in Table 4 and Figure.1.

The results showed that pinocembrin racemate could reduce the volume of cerebral infarction caused by ischemic cerebral stroke.

3. Determination of regional cerebral blood flow

The rats were fixed pronely on the stereotaxic apparatus and subjected to craniotomy. After cleaning for operation visual field, bregma was used as an origin, and a site situated 2mm behind and 3mm right to the bregma was selected as a measuring point. The region 2-3 cm around the site was thinned by a dental drill. In the process, the integrity of dura was maintained and large vessels were avoided. Probe holder was located and fixed.

The rats were supinely fixed on the operating table to carry out MCAO surgery. When a nylon line was inserted in ICA, it was not inserted into intracranial part until LDF value was stable, and blood flow value in 10 min was recorded, and an average value thereof is used as a baseline value of cerebral blood flow. After the MCAO surgery completed,

the nylon line was inserted into the intracranial part. When the blood flow value suddenly declined to 20-30% of the baseline value, it was noted that the blood flow in MCA had been blocked. The blood flow value before MCAO in every group was used as a baseline value (100%) of the group. The blood flow values after surgery were showed in percentages of the baseline value. LDF value was determined at the same site before the animals was put to death.

30 min later after the cerebral ischemia in rats, the rCBF value of the model group was (31.09±5.35)% of baseline value. The rCBF values in the pinocembrin racemate groups (3, 10, 30mg/kg) and the nimodipine group (3mg/kg) were respectively (40.76±6.58)%, (50.09±7.09)%, (53.28±8.03)% and (55.58±6.09)% of their baseline values. It can be seen that cerebral blood flows in all the administration groups recovered rapidly with a significant increase as compared with that of the model group, in which the pinocembrin racemate groups (10, 30mg/kg) and the nimodipine group (3mg/kg) showed significant difference as compared with the model group ($P<0.05$). Owing to the compensatory of collateral circulation, the extent of regional cerebral blood flow decreased gradually after ischemia occurring, but still kept at a level higher than that in the model group.

Table 5: Effects ofpinocembrin racemate on regional cerebral blood flow (rCBF) ($\bar{x}$ ± sd, n=10)

| Time point (min) | Sham operation group | Model group | Nimodipine | pinocembrin racemate 3 mg/kg | pinocembrin racemate 10mg/kg | pinocembrin racemate 30mg/kg |
|---|---|---|---|---|---|---|
| 10 | 99.91±7.2 7 | 99.91±7.27 | 99.91±7.27 | 99.91±7.27 | 99.91±7.27 | 99.91±7.27 |
| 20 | 98.89±6.9 2 | 31.61±6.58## | 32.98±8.09 | 32.03±6.09 | 31.45±5.6 | 37.98±6.87 |
| 30 | 97.76±7.5 1 | 31.09±5.35## | 55.58±6.09 * | 40.76±6.58 | 50.09±7.09* | 53.28±8.03* |
| 40 | 98.59±6.0 9 | 32.91±5.25## | 67.96±7.81 * | 41.59±6.35 | 47.72±6.67* | 54.92±8.58* |
| 50 | 98.81±6.3 5 | 33.28±7.09## | 66.59±9.08 * | 42.81±5.25 | 49.98±7.1* | 55.01±6.25* |
| 60 | 97.8±6.25 | 34.92±6.67## | 64.09±8.03 * | 40.8±7.09 | 48.99±8.14* | 57.95±6.09* |
| 70 | 97.69±6.0 9 | 35.01±7.1## | 65.72±8.58 * | 41.69±8.67 | 47.01±8.09 | 53.75±7.09* |
| 80 | 98.75±5.6 7 | 37.95±8.14## | 64.98±6.25 * | 40.75±7.1 | 46.07±9.08 | 54.99±6.67* |
| 90 | 98.99±6.1 | 36.82±8.09## | 63.99±6.09 * | 40.99±8.14 | 51.85±8.03* | 55.16±7.1* |
| 100 | 97.16±7.1 4 | 39.05±6.09## | 67.01±5.67 * | 41.16±6.25 | 47.12±8.58 | 56.59±8.14* |
| 110 | 98.59±8.0 9 | 43.56±7.81## | 63.07±6.1* | 46.59±7.09 | 46.89±6.25 | 57.8±7.27* |
| 120 | 97.8±6.81 | 45.07±9.08## | 61.85±7.14 * | 45.8±5.67 | 51.8±6.09 | 64.09±6.09* |
| 130 | 99.9±8.08 | 46.11±8.03## | 67.12±8.09 * | 46.99±6.1 | 56.59±9.08 | 61.72±6.58* |
| 140 | 99.86±9.0 3 | 45.99±8.58## | 65.89±6.81 * | 48.01±7.14 | 57.8±8.03 | 64.98±6.35 |
| 150 | 99.7±7.58 | 46.78±7.89## | 61.8±8.08* | 44.07±7.09 | 56.99±8.58* | 63.99±5.25 |
| 160 | 99.69±6.8 9 | 47.08±8.19## | 63.47±9.03 | 47.85±6.81 | 58.01±7.89 | 57.01±7.09 |
| 170 | 98.13±7.1 9 | 48.29±6.98## | 65.9±7.58* | 49.12±6.08 | 54.07±6.81 | 65.58±8.67* |

(continued)

| Time point (min) | Sham operation group | Model group | Nimodipine | pinocembrin racemate 3 mg/kg | pinocembrin racemate 10mg/kg | pinocembrin racemate 30mg/kg |
|---|---|---|---|---|---|---|
| 180 | 96.98±5.9 8 | 48.6±7.16## | 66.08±6.89 * | 49.89±7.58 | 55.9±6.08 | 62.96±6.81* |

Compared with the sham operation group, ##P<0.01; Compared with the model group, *P<0.05.

4. Cerebral edema and determination of Evans blue (EB) and fluorescein sodium (NF)

After surgery, a mixture solution of EB/NF (0.5%, dissolved in normal saline) of 0.25ml was immediately (immediately after administration in the administration groups) injected via tail vein in the animals. 24 hours later, a normal saline was perfused in the hearts of the rats to get rid of unbound dye. The rats were decollated and the brain tissues were promptly removed and separated into ischemic hemisphere and non-ischemic hemisphere. The two hemispheres were weighted respectively and homogenated with 7.5% (w/v) trichloroacetic acid (TCA). The homogenate was divided into two parts, in which 1ml homogenate was adjusted with 52μl NaOH (5N) to reach a neutral pH value, and 200μl of it was taken out to determine the fluorescence intensity (excitation 485nm, emission 535nm), the NF thereof was determined by using a standard curve made with NF solutions of concentration series. Another part of homogenate was centrifuged for 20 min at 12000 g and 4˚C. 200μl supernatant was taken out to a microplate and its absorbance was determined at 620nm, and EB thereof was determined by using a standard curve made with EB solutions of concentration series. The results were expressed in form of μg EB (or NF)/g brain wet weight. Percentage of brain edema was expressed as:

(brain weight in ischemic hemisphere - brain weight in non-ischemic hemisphere)/ brain weight in non-ischemic hemisphere×100%.

As Table 6 showed, the percentage of brain edema in the model group was (8.3±1.9)%, while the percentages of brain edema in the pinocembrin racemate groups (3mg/kg, 10mg/kg, 30 mg/kg, iv) were respectively (5.5±1.7)%, (4.1±1.5)%, (3.2±2.1)%, which showed significant difference as compared with the model group (p<0.05, p<0.01). Pinocembrin racemate (3mg/kg, 10mg/kg, 30 mg/kg, iv) also had significantly reduced the EB/NF leakage in the tissue. These results showed that pinocembrin could mitigate the tissue edema caused by cerebral ischemia.

Table 6: Effects of pinocembrin racemate on brain edema and EB/NF leakage in MCAO rats ($\overline{x}$ ± sd, n=6)

| Group | n | Percentage of brain edema volume (%) | EB leakage (μg/g tissue) | NF leakage (μg/g tissue) |
|---|---|---|---|---|
| model | 6 | 8.3±1.9 | 8.6±2.0 | 2.33±0.30 |
| pinocembrin racemate 3mg/kg | 6 | 5.5±1.7* | 6.16±0.4* | 1.48±0.08* |
| pinocembrin racemate 10mg/kg | 6 | 4.1±1.5* | 5.03±0.8* | 1.39±0.20* |
| pinocembrin racemate 30mg/kg | 6 | 3.2±2.1 | 4.39±0.4** | 1.30±0.15** |

Compared with the model group, *P<0.05, **P<0.01.

5. Determination of energy metabolism indexes in cerebral ischemic tissue in rats

The results were shown in table 7. After 24 hours of cerebral ischemia, the energy index in the model group decreased to be 42.6% of that before surgery (P<0.05). Compared with the model group, the energy index in pinocembrin racemate groups (3mg/kg, 10mg/kg, 30 mg/kg) increased significantly and increased respectively by 34.6% and 45.8% (P<0.05) relative to the model group, showing a dose-dependence.

Results in recovery test and repeatability test showed that the injection volumes of ATP, ADP and AMP had good linear relationship with peak areas. The obtained r values were respectively $r_{ATP}$=0.9897, $r_{ADP}$=0.9896, $r_{AMP}$=0.9893, $r_{CrP}$=0.9981. The recovery rates of 4 kinds of standard materials were respectively (86.6±5.6)%, (94.45±7.5)%, (83.4±6.1)%, (78.69±7.3)%.

Table 7: Effects of pinocembrin racemate on energy metabolism indexes in cerebral ischemic tissue in MCAO rats ($\bar{x}$ ± s, n=10)

| Group | | Sham operation | model | pinocembrin racemate | | |
|---|---|---|---|---|---|---|
| | | | | 3mg/kg | 10mg/kg | 30mg/kg |
| Parameter (μmol/g) | ATP | 2.55±0.45 | 1.01±0.12# | 1.18±0.18 | 1.62±0.21* | 1.97±0.58* |
| | ADP | 0.42±0.07 | 0.28±0.03 | 0.31±0.04 | 0.35±0.06 | 0.38±0.05 |
| | AMP | 0.01±0.00 | 0.02±0.00 | 0.02±0.00 | 0.02±0.00 | 0.02±0.00 |
| | CrP | 7.18±1.46 | 3.16±1.23# | 3.98±1.23* | 4.12±1.25* | 4.43±1.34* |
| Energy Load | | 1.54±0.33 | 0.66±0.23# | 0.73±0.23 | 0.88±0.23* | 0.96±0.29* |

Compared with the sham operation group, #P<0.05; Compared with the model group, *P<0.05.

6. Effects of pinocembrin racemate on inflammation in acute stage of cerebral ischemia (24 hours after ischemia)

6.1 NO and TNF-α contents in serum

It was showed in Table 8 that the NO and TNF-α contents in serum in the model group increased significantly, and in the pinocembrin racemate groups (3mg/kg, 10mg/kg, 30 mg/kg), the NO and TNF-α contents in rats' serums at the time 24 hours after ischemia decreased significantly (P < 0.05, P < 0.01).

Table 8: Effects of pinocembrin racemate on NO and TNF-α contents in MCAO rats' serum ($\bar{x}$ ± sd, n=8)

| Group | n | NO (μmol/L) | TNF-α (pg/ml) |
|---|---|---|---|
| Normal | 8 | 11.45±2.42 | 89.58±34.31 |
| Model | 8 | 127.30±12.35## | 1442.45±52.72## |
| pinocembrin racemate 3mg/kg | 8 | 109.80±9.38* | 1163.09±51.12* |
| pinocembrin racemate 10mg/kg | 8 | 107.11±9.13** | 1104.83±48.43** |
| pinocembrin racemate 30mg/kg | 8 | 104.75±5.03** | 1057.48±48.48** |

Compared with the normal group, #P<0.05, ##P<0.01; Compared with the model group, *P<0.05, **P<0.01.

6.2 Effects of pinocembrin racemate on expression of cytokines such as TNF-α, IL-1β in cerebral ischemia tissue

It can be seen in Figure 2 that the expression of cytokines of TNF-α and IL-1β in tissue increased significantly at the time 24h after ischemia, and the administration of pinocembrin racemate (3mg/kg, 10mg/kg, 30 mg/kg) in early stage of ischemia inhibited the expression of TNF-α, IL-1β in some extents.

6.3 Effects of pinocembrin racemate on expression of adhesion molecules such as ICAM-1, VCAM-1 in cerebral ischemic tissue

It can be seen in Figure 2 that the expression of adhesion molecules of ICAM-1 and VCAM-1 in tissue increased significantly at the time 24h after ischemia, and the administration of pinocembrin racemate (3mg/kg, 10mg/kg, 30 mg/kg) in early stage of ischemia inhibited the expression of TNF-α and IL-1β in some extents.

6.4 Effects of pinocembrin racemate on expression of iNOS and AQP-4 protein

It can be seen in Figure 2 that the expression of iNOS and AQP-4 protein in tissue increased significantly at

the time 24h after ischemia, and the administration of pinocembrin racemate (3mg/kg, 10mg/kg, 30 mg/kg) in early stage of ischemia inhibited the expression of iNOS and AQP-4 protein in some extents.

With β-actin as internal standard, the expressions of TNF-α, IL-1β, ICAM-1, VCAM-1, iNOS, AQP-4 were quantified, and the inhibition effects of pinocembrin racemate on the above proteins were seen intuitively. Pinocembrin racemate in doses of 10mg/kg and 30 mg/kg showed better results.

The above results showed that pinocembrin racemate could decrease acute inflammation caused by acute ischemic stroke.

7. Observations of neurons morphology in cerebral ischemia tissue in rats

After cerebral ischemia for 24 hours, the rats were anesthetized by intraperitoneal injection of 10% Chloral hydrate. After perfused via heart with heparinized normal saline for 10 min and 4% paraformaldehyde for 30 min, the brain tissue was taken out and moved in 4% paraformaldehyde to fix, then cut into coronal sections (6μm) by a frozen section machine, and one section was taken out in every 20 sections to stain. The stained sections were expanded on a slide glass treated by polylysine, and then preserved in a refrigerator at -40˚C.

Nissel staining: (1) the frozen section was taken out from the refrigerator and dried in room temperature; (2) put into acetone to fix for 30 min and washed by PBS for 3 times, 3 min every time; (3) dipped for 20-30 min in toluidine blue dye and then washed by water for 15min; (4) dehydrated by ethanol in gradient manner and vitrified by xylene and mounted by a neutral gum; (5) observed under a light microscope and photographed for analysis; (6) 4 frozen sections in roughly same position for each rat were taken out and 5 vision fields in hippocampus for each section were observed under 200x light microscope (totally 20 visions per rat). The cells in vision fields were counted. The averages of ratios of the numbers in the administration groups to the number in the model group were calculated for statistical analysis.

The results were shown Figure 3. Hippocampus is an area sensitive to cerebral ischemia. Nissel staining results showed that, after the cerebral tissue was ischemicaly injured, a serious damage in hippocampal neurons, an obvious absence of cells and a loose arrangement of nerve cells were observed. It was showed in quantitative results that nerve cells decreased 79.5±9.7% in comparison with the sham-operation group and the difference was significant (P<0.01). Pinocembrin (3mg/kg, 10mg/kg, 30 mg/kg) could improve the shape of ischemic nerve cells and decrease the loss of nerve cells, in which nerve cells increased by 11.5±8.9%, 36.8±4.9% and 51.7±6.6% in comparison with the model group, indicating significant differences (P<0.05) in comparison with the model group and dose-dependence, which enlightened neuroprotective activities after acute cerebral ischemia.

Summary:

1) Pinocembrin racemate can relieve behavioral changes caused by ischemic stroke;
2) Pinocembrin racemate can relieve decline of cerebral blood flow caused by ischemic stroke;
3) Pinocembrin racemate can reduce volume of cerebral infarction caused by ischemic stroke;
4) Pinocembrin racemate can relieve cerebral edema caused by ischemic stroke;
5) Pinocembrin racemate can relieve energy metabolism disorder caused by ischemic stroke;
6) Pinocembrin racemate can relieve acute inflammation caused by ischemic stroke;
7) Pinocembrin racemate can relieve nerve cell damage caused by ischemic stroke.

Example 3: Acute toxicity study and evaluation

Experimental results showed that, with single intravenous injection in SD rats, LD50 value of pinocembrin racemate was 490.9 (367.6~746.7) mg/kg, LD50 value of (S)-pinocembrin was 375.3 (271.2~538.5) mg/kg, and LD50 value of (R)-pinocembrin was 347.8 (257.4~466.3) mg/kg. The above results showed that pinocembrin racemate had a larger safe range for use. All these drugs had no effect to animal's weight, and their major apparences in term of toxicity were quadriplegic and mild blood stasis in liver and lung.

Example 4: Studies of therapeutic time window of pinocembrin in focal cerebral ischemia-reperfusion injured rats

1. In the test, transient middle cerebral artery occlusion (tMCAO) model of rats was adopted. The reperfusion was performed after focal cerebral ischemia for 2 hours so as to inspect therapeutic time window of pinocembrin after focal cerebral ischemia. The rats were administered by pinocembrin racemate (1 mg/kg, 5 mg/kg, intravenous

injection) after reperfusion 1 hour, 4 hours and 6 hours (i.e., ischemia 3 hour, 6 hours and 8 hours). (R)-pinocembrin and (S)-pinocembrin (1 mg/kg, 5 mg/kg, intravenous injection) were administered after reperfusion 1 hour and 4 hours (i.e., ischemia 3 hour and 6 hours). The therapeutic time windows of these drugs in focal cerebral ischemia were assessed by studying their effects to neurological symptom scores, infarct sizes and brain water contents during 24 hours after tMCAO.

2. Materials and methods:

2.1 Experimental animals:

100 male SD rats, 240-280 g, purchased from Beijing Weitong Lihua Experimental Animal Technology Co., Ltd. Certificate of Conformity: SCXK ( JING ) 2007-0001. The animals were raised in a routine way before and after surgery at 23-25˚C with free supply of food and water.

2.2 Drugs and reagents:

Pinocembrin racemate for injection, (R)-pinocembrin for injection and (S)-pinocembrin for injection were provided by the New Drug Development Laboratory in the Drug Research Institute of the Chinese Academy of Medical Sciences (Batch number: 20050601, content: 2.36%), and prepared by the method disclosed in CN200810084682.3, i.e., by forming inclusion complexes of pinocembrin racemate or enantiomers of pinocembrin with cyclodextrin or its derivatives, and dissolving the complexes in physiological saline when used. Nimodipine as positive control drug was purchased from Bayer Company (Germany). Hydroxypropyl β-cyclodextrin was provided by the New Drug Development Laboratory in the Drug Research Institute of the Chinese Academy of Medical Sciences. TTC was purchased from Sigma Company. Other reagents were commercially available reagents of analytical grade.

2.3 Preparation of tMCAO model

Rat anesthesia: 400 mg/kg 10% chloral hydrate was intraperitoneally injected in rats and then righting reflex disappeared.

The rats were supinely fixed on an operating table and subjected to anterior neck cut and blunt separation of layers of organization to expose right common carotid artery (CCA). CCA was separated to the segment after bifurcation of internal carotid artery (ICA) and external carotid artery (ECA). Injury to vagus nerve and trachea was avoided. Lines were placed under the CCA and ECA for standby use.

The CCA and ICA were clipped with artery clips. Two No.0 surgery lines were litigated on the ECA distal end with 2~3mm spacing and then the vascular between the two surgery lines was cut. The distal end of ECA was pulled until it formed a straight line with the ICA. An incision was cut on ECA and a nylon line was inserted through ECA into ICA. After the artery clip was loosen, the nylon line was pushed on into the brain through ICA and inserted in depth of 18.5±0.5mm when resistance was felt slightly. The nylon line reached a smaller anterior cerebral artery through the beginning of MCA and then the blood flow obstruction in right MCA was established. the animals were kept in anesthesia during the obstruction. After 2 hours of obstruction, the nylon line was gently pulled out to ECA stub to form reperfusion. During the surgery process, the animals were irradiated by table lamp of 100W to maintain the body temperature. The room temperature was kept in range of 23-25˚C.

The rats in the sham operation group were subjected to only preoperative anesthesia and vascular dissection without ligation and importing line.

2.4 Experimental grouping and administration

Sham operation group (intravenous injection of normal saline after 3 hours of ischemia);

Model group (intravenous injection of 50 mg/kg hydroxypropyl β-cyclodextrin after 3 hours of ischemia);

Nimodipine group (1 mg/kg, administered after 3 hours of ischemia);

Pinocembrin racemate group (1 mg/kg, 5 mg/kg, administered after 3, 6 and 8 hours of ischemia);
(R)- pinocembrin group (1 mg/kg, 5 mg/kg, administered after 3 and 6 hours of ischemia);
(S)- pinocembrin group (1 mg/kg, 5 mg/kg, administered after 3 and 6 hours of ischemia).

First administration was performed by intravenous injection, and intraperitoneal injection was carried out once

12 hours later with a dose 1.5 times of that for intravenous injection.

2.5 Neurobehavioral examination

Neurobehavioral examination was performed respectively when the animals were awake after surgery and before the animals were put to death. Bederson's score was adopted (see details in Example 2, 1. Neurobehavioral Examination).

2.6 Determination of infarct volume:

After neurobehavioral scoring, the rats were put to death by decollation and their brain tissues were taken out quickly and put into a refrigerator of -20˚C. 10 min later, the brain tissues were moved to an environment of room temperature. After the olfactory bulb, cerebellar and lower brain stem were resected, the brain tissue was cut into six continuous coronal slices with an interval of 2mm. Then the brain slices were quickly put into 5ml solution containing 2% TTC and incubated at a constant temperature of 37˚C and in a condition of darkness for 30min. During the time, the brain slices were tipped every 5 min. After TTC staining, normal tissue showed rose red color, while the infract tissue was not stained and showed white color. Every group of brain slices were put in order and photographed. The photographs were processed by an image analysis system and the infarct areas in each slice were calculated. The infarct volume of each slice was calculated via multiplying the infarct area by thickness of slice (2mm), and the infarct volume was the sum of infarct volumes of all slices. The infarct volume percentage was expressed as: sick-side infarct volume / total sick-side brain volume.

2.7 Determination of brain water content

The wet weight of each animal's brain was weighted before sliced. After staining, the brain slices were dried at 105˚C for 24 hours, and the dry weight of each animal's brain was weighted. The brain water contents of animals of various groups were compared. Brain water content = (brain wet weight - brain dry weight) / brain wet weight × 100%.

2.8 Data Processing:

The data of quantitative measurements were expressed as mean ± SD for t test. The count values were expressed as % for $X^2$ test.

3. Experimental results

3.1 Effects ofpinocembrin on Bederson's Value in tMCAO rats

The neurobehavioral score in the sham operation group was 0. The average score of rats in the solvent control group was 3.4±0.6, in which most animals showed internal rotation or retraction of their forelimbs of surgery-contralateral side, decrease of muscle stretching force of contralateral side or circling, and were scored 3; a minority of animals only showed internal rotation of their forelimbs and decrease of resistance, and were scored 2; and several animals showed severe symptoms and did not move by themselves, were scored 4.

In the pinocembrin racemate groups (1mg/kg, 5mg/kg), the symptoms of nerve injury after 3 hours and 6 hours of ischemia in animals were improved significantly (P<0.05 ,P<0.01), while the improvement to symptoms after 8 hours of ischemia was not significant, showing dose-dependence in some extent. In the (R)-pinocembrin groups and the (S)-pinocembrin groups (1mg/kg, 5mg/kg), the symptoms of nerve injury after 3 hours of ischemia were improved significantly (P<0.05), while the improvement to symptoms after 6 hours of ischemia was not significant. Furthermore, nimodipine (1 mg/kg) showed no improvement to symptoms after 3 hours of ischemia.

3.2 Effects ofpinocembrin on infarct volume in tMCAO rats

After 24 hours of tMCAO in rats, the consequent brain coronary slices were stained by TTC. There were infarct areas (white) that had not been stained in a certain percentage of animals in each group, indicating that animals automatically restored more or less within 24 hours of reperfusion. In the statistical process, the samples which infarct volume was less than 5% were removed from each group. Pinocembrin racemate (1 mg/kg) reduced infarct volume when initially administered after 3 hours of cerebral ischemia, and its activity was not significant when initially administered after 6 hours of cerebral ischemia. Pinocembrin racemate (5 mg/kg) reduced infarct volume when initially administered after 3 hours and 6 hours of cerebral ischemia (P<0.05, P<0.01), and its activity was not significant when initially administered

after 8 hours of cerebral ischemia. These effects showed a dose-dependence in some extents. (R)-pinocembrin and (S)-pinocembrin (1 mg/kg, 5 mg/kg) reduced the infarct volume after 3 hours of cerebral ischemia (P<0.05) and their activities were not significant when initially administered after 6 hours of cerebral ischemia. Furthermore, nimodipine (1 mg/kg) didn't show improvement when initially administered after 3 hours of cerebral ischemia.

3.3 Effects ofpinocembrin on cerebral edema in tMCAO rats

In the model group, brain water content of rats increased significantly. Pinocembrin racemate (1 mg/kg, 5 mg/kg) reduced significantly the brain water content after 3 hours and 6 hours of cerebral ischemia (P<0.05, P<0.01) and its activities were not significant when initially administered after 8 hours of cerebral ischemia. These activities were dose-dependent in some extents. (R)-pinocembrin and (S)-pinocembrin (1 mg/kg, 5 mg/kg) reduced brain water content after 3 hours of cerebral ischemia (P<0.05) and their activities were not significant when initially administered after 6 hours of cerebral ischemia. Furthermore, nimodipine (1 mg/kg) didn't show improvement when initially administered after 3 hours of cerebral ischemia. The results are shown in Table 9.

Table 9: Effects ofpinocembrin on neurobehavioral score, infarct volume and brain water content in rats after 2 hours of ischemia and 24 hours of reperfusion ($x \pm s$)

| Group | | n | Neurobehavioral value | Infarct percentage (%) | Brain water content (%) |
|---|---|---|---|---|---|
| Sham-operation | | 6 | 0 | 0 | 80.86±0.83 |
| Model | | 11 | 2.36±0.81## | 30.62±17.06## | 83.86±1.18## |
| Nimodipine,1mg/kg, 3h | | 11 | 2.09±0.63 | 31.30±10.31 | 83.33±1.01 |
| pinocembrin racemate | 1mg/kg, 3h | 9 | 1.44±0.72* | 16.59±7.98* | 82.20±1.00* |
| | 1 mg/kg, 6h | 11 | 1.68±0.46* | 23.01±11.00 | 82.44±1.28* |
| | 1 mg/kg, 8h | 12 | 1.88±0.61 | 27.16±13.69 | 83.26±1.05 |
| | 5 mg/kg, 3h | 9 | 1.44±0.52** | 13.64±6.32* | 82.23±1.42* |
| | 5 mg/kg, 6h | 12 | 1.66±0.77* | 15.03±4.07* | 81.12±1.42** |
| | 5 mg/kg, 8h | 12 | 1.91±0.66 | 28.32±18.65 | 83.29±1.79 |
| (R)-pinocembrin | 1 mg/kg, 3h | 9 | 1.42±0.82* | 15.69±7.82* | 82.18±0.98* |
| | 1 mg/kg, 6h | 11 | 1.86±0.57 | 26.33±12.67 | 83.36±1.12 |
| | 5 mg/kg, 3h | 9 | 1.39±0.72* | 15.66±7.88* | 81.20±1.03* |
| | 5 mg/kg, 6h | 12 | 1.83±0.49 | 25.89±13.57 | 83.77±1.07 |
| (S)-pinocembrin | 1 mg/kg, 3h | 9 | 1.29±0.72* | 15.32±7.98* | 81.95±1.00* |
| | 1 mg/kg, 6h | 11 | 1.79±0.63 | 28.06±12.98 | 82.96±0.98 |
| | 5 mg/kg, 3h | 9 | 1.59±0.77* | 15.78±7.28* | 81.87±1.12* |
| | 5 mg/kg, 6h | 12 | 1.77±0.70 | 25.97±12.75 | 83.37±1.27 |

Compared with the sham-operation group, ##P<0.01; compared with the model group, *P<0.05, **P<0.01.

4. Conclusion:

The results showed that the injection of pinocembrin racemate (1 mg/kg, 5 mg/kg) after 3 hours and 6 hours of ischemia decreased neurobehavioral injury and reduced infarct volume and mitigated cerebral edema. The activities were significant and showed a good dose-effect relationship. (R)-pinocembrin and (S)- pinocembrin showed significant effects when initially injected after 3 hours of ischemia in rats but were not effective when initially injected after 6 hours of ischemia. Nimodipine was not significantly effective in the experimental conditions. Pinocembrin racemate (1 mg/kg, 5 mg/kg, iv) had good therapeutic effects to acute ischemic stroke in rats, and the therapeutic time window thereof was about 6 hours.

## Claims

1. Use of a racemate of pinocembrin, a racemate of pinocembrin salt, a racemate of pinocembrin precursor or a racemate of pinocembrin hydrate in manufacture of a medicament for treatment of stroke.

2. The use according to claim 1, **characterized in that** the stroke is acute ischemic stroke.

3. A pharmaceutical composition for treatment of stroke, consisting of a racemate of pinocembrin, a racemate of pinocembrin salt, a racemate of pinocembrin precursor or a racemate of pinocembrin hydrate and a pharmaceutically acceptable excipient.

4. Use of (R)-pinocembrin or a salt, precursor or hydrate thereof in manufacture of a medicament for prophylaxis and treatment of stroke.

5. The use according to claim 4, **characterized in that** the stroke is acute ischemic stroke.

6. A pharmaceutical composition for prophylaxis and treatment of stroke, consisting of (R)-pinocembrin or a salt, precursor or hydrate thereof and a pharmaceutically acceptable excipient.

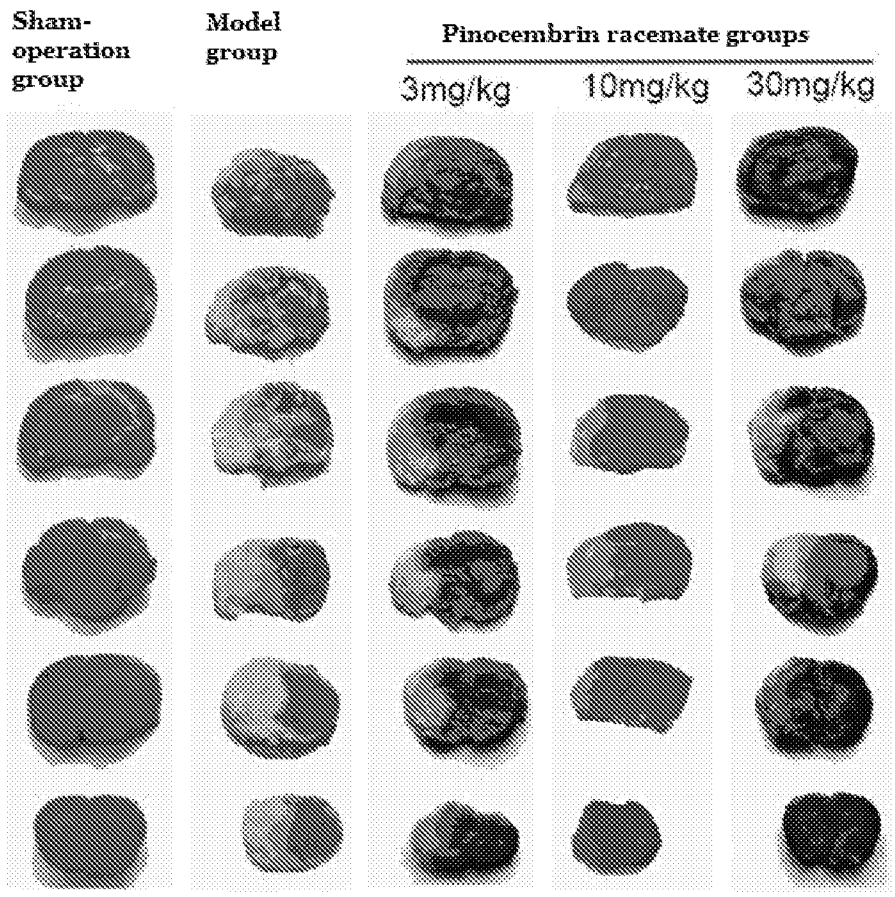
Sham-operation group
Model group
Pinocembrin racemate groups
3mg/kg
10mg/kg
30mg/kg

Figure 1

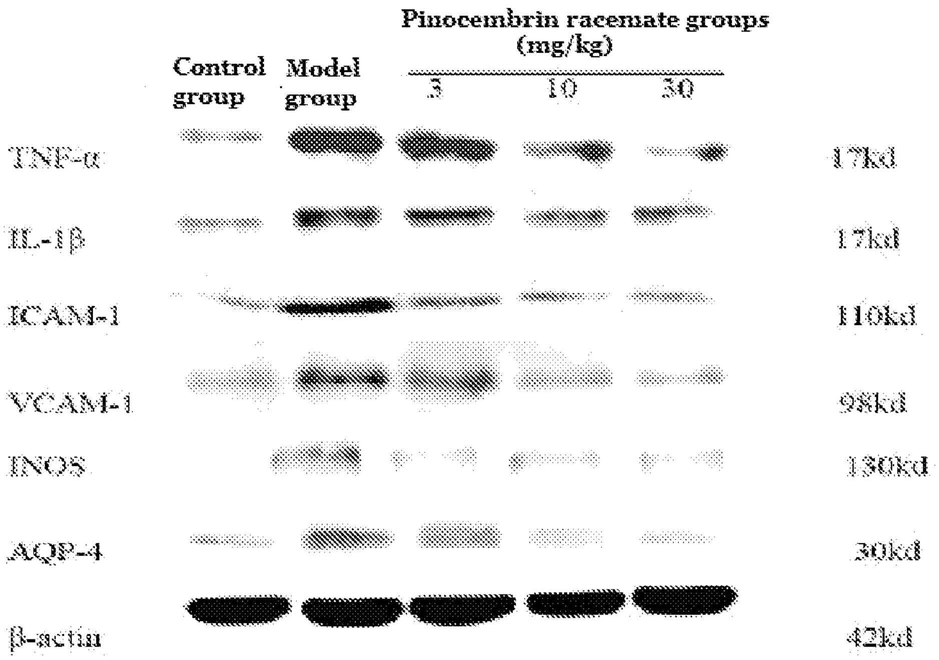


# Figure 2-A

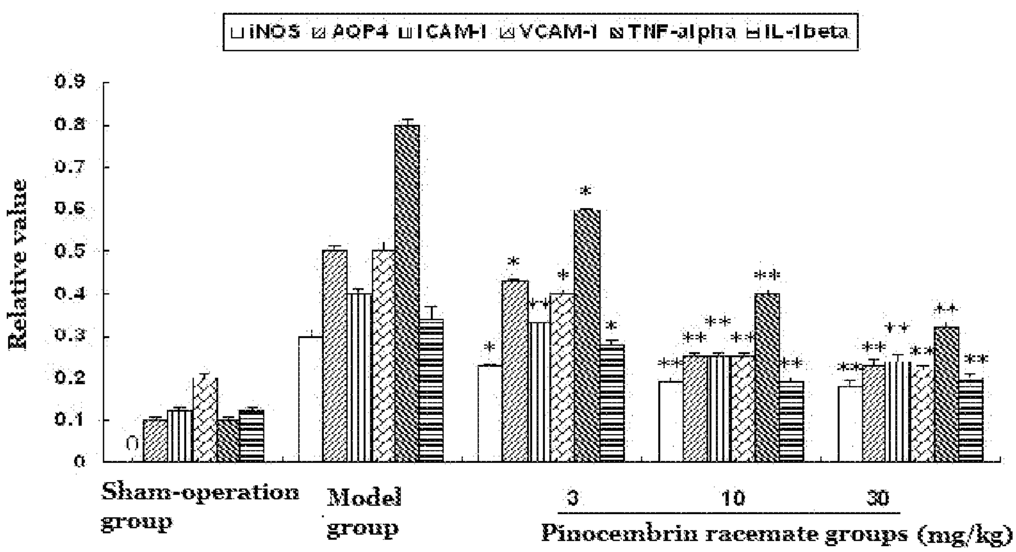


# Figure 2-B

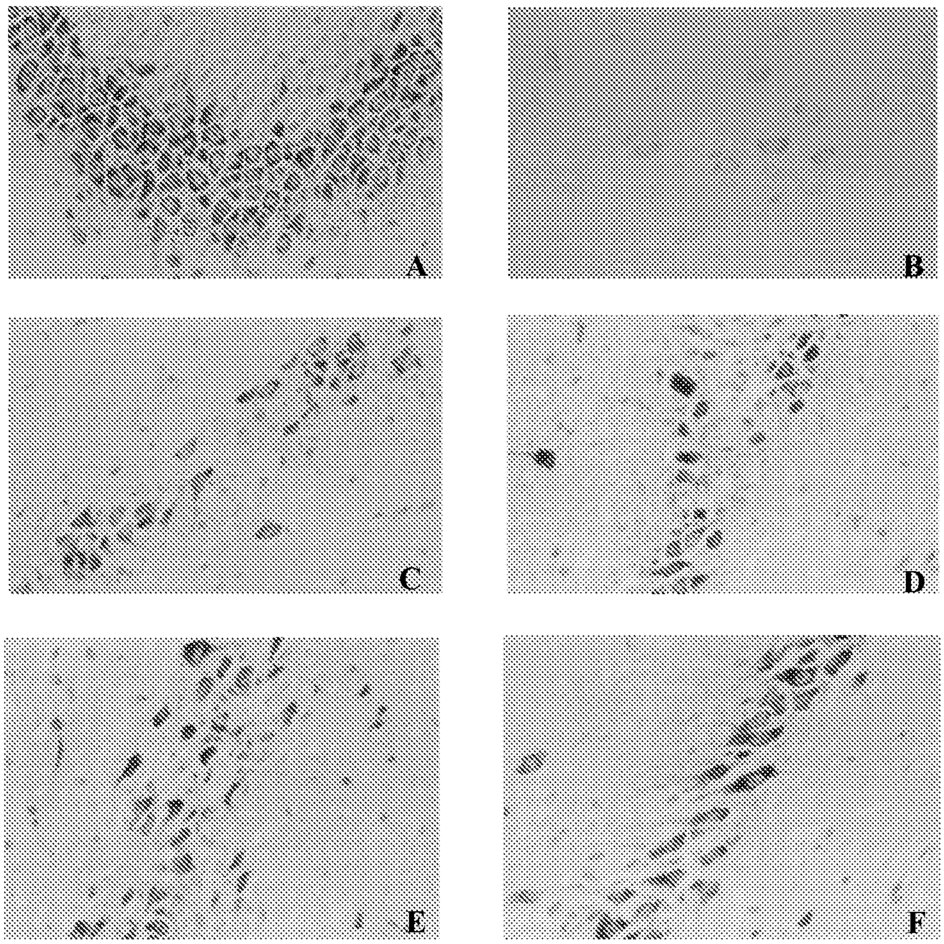


Figure 3

# INTERNATIONAL SEARCH REPORT

International application No.
PCT/CN2009/075456

**A. CLASSIFICATION OF SUBJECT MATTER**

See the extra sheet

According to International Patent Classification (IPC) or to both national classification and IPC

B. FIELDS SEARCHED

Minimum documentation searched (classification system followed by classification symbols)

IPC: A61K, A61P

Documentation searched other than minimum documentation to the extent that such documents are included in the fields searched

Electronic data base consulted during the international search (name of data base and, where practicable, search terms used)

CNPAT(Cprs), CTCMPD , CNKI Full-Text database, Chinese Medicine Abstract, WPI, EPODOC, PAJ, CA, Embase;
pinocembrin, propolis, honey, stroke, apoplexy, acute, ischemia, acacia, eucalyptus

C. DOCUMENTS CONSIDERED TO BE RELEVANT

| Category* | Citation of document, with indication, where appropriate, of the relevant passages | Relevant to claim No. |
|---|---|---|
| E | CN101537188A (Institute of Materia Medica Chinese Academy of Medical Sciences), 23 Sep. 2009(23. 09. 2009), see the whole document | 1-3 |
| X | GAO Mei et al. Acute neurovascular unit protective action of pinocembrin against permanent cerebral ischemia in rats. J Asian Nat Prod Res. 2008 May-June; 10(5-6); see the abstract | 1-6 |
| X | CN1695608A (Institute of Materia Medica Chinese Academy of Medical Sciences), 16 Nov. 2005(16. 11. 2005), see claims 1, 3, and description page 2 the penultimate line 5-page 3 line 2 | 1-3 |
| A | ------ | 4-6 |

☐ Further documents are listed in the continuation of Box C. ☒ See patent family annex.

* Special categories of cited documents:

"A" document defining the general state of the art which is not considered to be of particular relevance

"E" earlier application or patent but published on or after the international filing date

"L" document which may throw doubts on priority claim (S) or which is cited to establish the publication date of another citation or other special reason (as specified)

"O" document referring to an oral disclosure, use, exhibition or other means

"P" document published prior to the international filing date but later than the priority date claimed

"T" later document published after the international filing date or priority date and not in conflict with the application but cited to understand the principle or theory underlying the invention

"X" document of particular relevance; the claimed invention cannot be considered novel or cannot be considered to involve an inventive step when the document is taken alone

"Y" document of particular relevance; the claimed invention cannot be considered to involve an inventive step when the document is combined with one or more other such documents, such combination being obvious to a person skilled in the art

"&"document member of the same patent family

| Date of the actual completion of the international search | Date of mailing of the international search report |
|---|---|
| 01 Mar. 2010(01. 03. 2010) | **25 Mar. 2010 (25.03.2010)** |

| Name and mailing address of the ISA/CN | Authorized officer |
|---|---|
| The State Intellectual Property Office, the P.R.China<br>6 Xitucheng Rd., Jimen Bridge, Haidian District, Beijing, China 100088<br>Facsimile No. 86-10-62019451 | CHEN Yanyan<br>Telephone No. (86-10)62411200 |

Form PCT/ISA /210 (second sheet) (July 2009)

**INTERNATIONAL SEARCH REPORT**
Information on patent family members

International application No.
PCT/CN2009/075456

| Patent Documents referred in the Report | Publication Date | Patent Family | Publication Date |
|---|---|---|---|
| CN101537188A | 23. 09. 2009 | NONE | |
| CN1695608A | 16. 11. 2005 | CN1285334C | 22. 11. 2006 |

Form PCT/ISA /210 (patent family annex) (July 2009)

## INTERNATIONAL SEARCH REPORT

International application No.
PCT/CN2009/075456

CLASSIFICATION OF SUBJECT MATTER:

A61K 31/352(2006. 01)i
A61K 9/00(2006. 01)i
A61K 9/08(2006. 01)i
A61P 9/10(2006. 01)i

Form PCT/ISA /210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 200810185559
- CN 200410037860
- CN 200810084682


### Non-patent literature cited in the description


- *Brain Res.,* 24 June 2008, vol. 1216, 104-15
- **Yonghao Cheng ; Yabo Duan ; Yan Qi.** Synthesis of 5,7-dihydroxy-flavanone. *Chemical Reagents,* 2006, vol. 28 (7), 437
- Medicinal Chemistry. Higher Education Press, 25-26
- **Zhu XM ; Fang LH ; Li YJ ; Du GH.** Endothelium-dependent and Endothelium-independent relaxation induced by pinocembrin in rat aortic rings. *Vascul Pharmacol.,* 2007, vol. 46 (3), 160
- **Mei Gao ; Wen-cui Zhang ; Qing-shan Liu ; Juan-juan Hu ; Geng-tao Liu ; Guan-hua Du.** Pinocembrin prevents glutamate-induced apoptosis in SH-S Y5Y neuronal cells via bax/bcl-2 ratio decrease. *Eur J Pharmacol.,* 2008, vol. 591 (1-3), 73-9
- Acute neurovascular unit protective action of pinocembrin against permanent cerebral ischemia in rats. *J Asian Nat Prod Res.,* May 2008, vol. 10 (5-6), 551-8
- Protections of pinocembrin on brain mitochondria contribute to cognitive improvement in chronic cerebral hypoperfused rats. *Eur J Pharmacol.,* 07 August 2006, vol. 542 (1-3), 77-83